# EUROPÄISCHE PATENTANMELDUNG

(11) **EP 2 116 229 A1**
(43) Veröffentlichungstag der Anmeldung: **11.11.2009**
(21) Anmeldenummer: 09003414.1
(22) Anmeldetag: 09.03.2009
(51) Int. Cl.: A61K 9/107

(54) **Kolloidales Trägersystem**

(30) Priorität: 18.03.2008 DE 102008015299
(71) Anmelder: PKH GmbH Halle, 06120 Halle (DE)
(72) Erfinder: Neidhardt, Hilltrud, 06120 Halle (DE); Neidhardt, Axel, 06120 Halle (DE)
(74) Vertreter: Donath, Dirk

(57) **Zusammenfassung**

Die Erfindung betrifft ein kolloidales Trägersystem, insbesondere ein stabiles, physiologisches, mischmizellares System in Form einer Mikro- oder Nanoemulsion sowie dessen Herstellung und Verwendung.

Das kolloidale Trägersystem umfasst eine Tensid/Cotensid - Mischung, eine hydrophile Phase und eine lipophile Komponente. Zur Herstellung des kolloidalen Trägersystems werden nichtionische Tenside, sowie lipophile und hydrophile Grundstoffe und Mischungen zusammengeführt, so dass sich die Mikroemulsion spontan aus den Einzelbestandteilen ausbildet.
Die Mikroemulsion wird zur dermalen und transdermalen Applikation schlecht resorbierbare Vitamine wie bspw. Ascorbinsäure, Biotin, Retinsäure, Thiamin, Calciferol und Tokopherol verwendet.

## Beschreibung

Die Erfindung betrifft ein kolloidales Trägersystem, insbesondere ein stabiles, physiologisches, mischmizellares System in Form einer Mikro- oder Nanoemulsion sowie dessen Herstellung und Verwendung.
Mikroemulsionen gehören seit geraumer Zeit zum Stand der Technik.
Kolloidale Systeme (Wirkstoffträger) werden derzeit in unterschiedlicher Weise eingesetzt. So existieren bspw. zur Anwendung von Liposomen als Arzneistoffträger umfangreiche Untersuchungen. Dabei kommen die kolloidalen Systeme sowohl topisch als auch systemisch zur Anwendung.
Aus DE 10 2004 004 394 A1 ist bspw. eine vorzugsweise optisch klare, transparente oder durchscheinende Mikroemulsion mit einem Gehalt an 20 bis 60 Gew.-% Wasser, 3 bis 20 Gew.-% einer Ölphase bekannt, welche im wesentlichen aus bei 25°C flüssigen, hydrophoben Ölen besteht, 20 bis 60 Gew.-% einer Emulgatormischung, welche im wesentlichen besteht aus mindestens einem ersten Emulgator, der ausgewählt ist aus hydrierten und nicht hydrierten ethoxylierten Rizinusölen, und mindestens einem zweiten Emulgator, welcher einen NRU₅₀-Wert von größer 110 µg/ml aufweist. Die Mikroemulsion ist als kosmetisches, dermatologisches oder pharmazeutisches Mittel anwendbar, insbesondere als Mittel zur Haarbehandlung.
DE 699 01 567 T2 offenbart eine einzunehmende Zusammensetzung mit einem System, das bei Kontakt mit einer nach Einnahme durch die physiologische Flüssigkeit gelieferten hydrophilen Phase selbstmikroemulgierbar ist, wobei das genannte System wenigstens einen Wirkstoff, eine lipophile Phase (bestehend aus einer Mischung aus Mono-, Di- und Triglyceriden mit C8-C18-Fettsäuren und aus Polyethylenglykolmono- und -diestern, die eine hydrophil-lipophile Balance [HLB] kleiner 16 aufweisen), ein Tensid (TA) auf Basis von Glyceriden mit einer HLB kleiner 16 (enthaltend gesättigte, polyglykolisierte C8-C10-Glyceride und Oleinester des Polyglyzerin, ein Co-Tensid (CoTA) ausgewählt aus der Gruppe enthaltend Fettsäureester des Propylenglykol, Oleinester des Polyglyzerin und Ethyldiglykol, wobei das Verhältnis TA zu CoTA zwischen 0,5 und 6 liegt.
Aus DE 39 30 928 A1 sind pharmazeutische Zusammensetzungen, die ein Cyclosporin (wie bspw. Ciclosporin oder [Nva]²-CiClosporin) in Form eines so genannten Mikroemulsions-vorkonzentrats oder einer Mikroemulsion enthalten, bekannt. Diese Zusammensetzungen enthalten gewöhnlich als Komponente 1.1. einen C₁-C₅-Alkyl- oder Tetrahydrofurfuryldiether oder -teilether eines niedermolekularen Mono-oder Polyoxyalkandiols, wie Transcutol oder Glycofurol, als hydrophile Komponente. Ferner gehören hierzu auch Zusammensetzungen, die ein Cyclosporin und eine Komponente 1.1. sowie einen Saccharidmonoester (wie bspw. Raffinosemonolaurat oder Saccharosemonolaurat) enthalten. Geeignete Dosierungsformen sind topische Formulierungen und insbesondere orale Dosierungsformen.
DE 100 26 678C1 offenbart ein äußerlich anzuwendendes Pflaster für einen entzündungshemmenden Wirkstoff, der eine nichtsteroide, entzündungshemmende Wirkstoffkomponente enthält, das hinsichtlich der dermalen Absorption und der Haftung an der Haut ausgezeichnete Eigenschaften aufweist und kaum zu einer Hautreizung führt. Die aus der nichtsteroiden, entzündungshemmenden Wirkstoffkomponente und einem hydrophilen, nichtionischen, oberflächenaktiven Mittel (Surfaktant) bestehende nichtsteroide, entzündungshemmende Wirkstoffzubereitung wurde in der aus wasserlöslichem Polymerenmaterial, wasserlöslichem Vinylpolymer und wasserunlöslichem mehrwertigen Metallsalz bestehende wasserhaltige Basis fein verteilt, um das äußerlich anzuwendende Pflaster zu schaffen, das den nichtsteroiden Wirkstoff im Zustand einer Mikroemulsion in der wasserhaltigen Basis gelöst und fein verteilt enthält, eine ausgezeichnete dermale Absorption ergibt und eine gute Haftung an der Haut aufweist, wobei es kaum Hautirritationen hervorruft.
DE 39 08 047 1 offenbart eine hochdisperse pharmazeutische Zusammensetzung, die als Mikroemulsion vorliegt und durch Mischen von mit Ionenpaarbildnern modifiziertem sauren oder basischen Wirkstoff, Isopropylmyristat als Fettkomponente, Polyoxyethylen-20-sorbitanmonooleat, Polyoxyethylen-20-Sorbitanmonolaurat und/oder Polyoxyethylen-30-stearylalkoholether als Tensid, Polyoxyethylen-7-glycerylcocoat als Cotensid und Wasser erhältlich ist. Die Zusammensetzung weist hohe Wirkstoffkonzentrationen auf und ist insbesondere zur nasalen, rektalen und transdermalen Applikation geeignet.

Die bisher bekannten Mikroemulsionen haben den Nachteil, dass insbesondere keine stabilen, physiologischen, mischmizellaren Formulierungen mit schlecht resorbierbaren Vitaminen realisierbar sind. Aufgabe der vorliegenden Erfindung ist es, ein kolloidales Trägersystem, insbesondere ein stabiles, physiologisches, mischmizellares System in Form einer Mikro- oder Nanoemulsion sowie ein Verfahren zu dessen Herstellung, die aufwandgering realisiert werden können, und deren Anwendung anzugeben, wobei insbesondere die Nachteile des Standes der Technik, vorallem hinsichtlich der schlecht resorbierbaren Vitamine (wie bspw. Ascorbinsäure, Biotin, Retinsäure, Thiamin, Calciferol und Tokopherol) bei der dermalen und transdermalen Applikation in der medizinischen Praxis zu vermeiden.

Erfindungsgemäß wird diese Aufgabe durch die kennzeichnenden Merkmale des ersten Patentanspruchs gelöst. Weitere günstige Ausgestaltungsmöglichkeiten der Erfindung sind in den nachgeordneten Patentansprüchen angegeben.

Das Wesen der Erfindung besteht darin, dass ein kolloidales Trägersystem, umfassend eine Tensid / Cotensid - Mischung, eine hydrophile Phase (bspw. in Form einer Mischung von Propylenglycol und Wasser) und eine lipophilen Komponente (bspw. Pelemol® BIP) bereit gestellt wird, das zur dermalen (topischen) Applikation von schlecht resorbierbaren Vitamine (wie bspw. Ascorbinsäure, Biotin, Retinsäure, Thiamin, Calciferol und Tokopherol, Dexpanthenol, D,L-α-Tocopherolacetat und Ascorbylpalmitat) verwendet werden kann.

Die mit dem erfindungsgemäßen kolloidalen Trägersystem verabreichbaren Salze der Pantothensäure bzw. Dexpanthenol wirken entzündungshemmend, granulationsfördermd sowie erythemlindernd und regen die mitotische Aktivität an.
Von besonderem Interesse in Bezug auf den so genannten oxidativen Stress sind die mit dem erfindungsgemäßen kolloidalen Trägersystem verabreichbaren Antioxidantien Ascorbinsäure und Tocopherol bzw. deren Derivate. Diese können die Auswirkungen reaktiver Sauerstoffspezies auf die Haut wie Entzündungen, Hautalterung oder Hautkrebsentstehung positiv beeinflussen. Außerdem ist Ascorbinsäure an der Bildung von Kollagen beteiligt und trägt so zur Aufrechterhaltung der mechanischen Eigenschaften der Haut bei.

Das erfindungsgemäße kolloidale Trägersystem erlaubt dermal anwendbare Formulierungen herzustellen, welche die stabile Inkorporierung der genannten Vitamine in therapeutisch relevanten Konzentrationen zulässt.

Diese Formulierungen zur topischen Applikation auf der Haut vermeiden die Nachteile des Standes der Technik, insbesondere hinsichtlich der schlecht resorbierbaren Vitamine (wie bspw. Ascorbinsäure, Biotin, Retinsäure, Thiamin, Calciferol und Tokopherol) bei der dermalen und transdermalen Applikation in der medizinischen Praxis.

Das erfindungsgemäße kolloidale Trägersystem weist dabei penetrationsfördernde Eigenschaften und eine gute Solubilisierungskapazität für lipophile und hydrophile Substanzen auf.

Das erfindungsgemäße kolloidale Trägersystem wird nachstehend an Hand der Ausführungsbeispiele erläutert, ohne dadurch auf diese beschränkt zu werden

### 1. Zusammensetzung und Herstellung des Trägersystems

**Tab. 1: Zusammensetzung der optimierten Mikroemulsionssysteme**

| | **Zusammensetzung** **(alle Prozentangaben in m/m)** | |
|---|---|---|
| **Myritol** | Tagat® 02/Synperonic® PE/L 101 (2:3) | 20% |
| | Myritol® G 318 | 5% |
| | Glycerol/Propylenglycol/Wasser (0,3:1:1) | 75% |
| **Myritol** | Tagat® 02/Synperonic® PE/L 101 (2:3) | 20% |
| | Myritol® G 318 | 5% |
| | Glycerol/Propylenglycol/Wasser (0,15:1:1) | 75% |

Mikroemulsionen bilden sich spontan aus den Einzelbestandteilen.
Dazu gehören nichtionische Tenside, sowie lipophile und hydrophile Grundstoffe und Mischungen.
Das Tensid/Cotensid-Gemisch besteht aus einer Kombination dermal gut verträglicher Substanzen unterschiedlicher HLB-Werte:

### Synperonic® PE L.101 /

Synperonic® PE L101 ist Handelsnamen für nichtionische Tenside aus der Gruppe der Poloxamere. Die Eigenschaften dieser Blockpolymere lassen sich durch die Synthese von Produkten mit unterschiedlichen Polyoxyethylen- und polyoxypropylen-Verhältnissen variieren. Synperonic® PE L101 (Poloxamer 331, Mᵣ∼ 3800), mit seinem niedrigeren Polyoxypropylen-Anteil, hat einen HLB-Wert von 1 und bei 25 °C eine Viskosität von 800 mPa•s. Anwendung finden die geschmacklosen, haut- und schleimhautverträglichen Substanzen als Emulgatoren für halbfeste Zubereitungen und auf Grund ihrer sehr geringen Hämolyseaktivität in parenteralen Fettemulsionen [4, 5].

### Tagat® 02

Tagat® 02 (Polyoxyethylenglycerolmonooleat) ist ein gelbes, flüssiges, nichtionisches Tensid mit einem HLB-Wert von 15,0. Es hat einen schwachen Eigengeruch, ist löslich in Wasser, dagegen in Fetten und Paraffinöl unlöslich. Die gut haut- und schleimhautverträgliche Substanz wird als O/W-Emulgator und Solubilisierungsmittel für polare Öle und Vitamine eingesetzt [6, *7]*.

### Myritol® G318

Myritol® G 318, auch als Miglyol® 812 N oder Neutralöl bezeichnet, ist ein Gemisch mittelkettiger Triglyceride. Zu mindestens 95 % enthält diese farblose, niedrigviskose, ölige Flüssigkeit die gesättigten Fettsäuren Capryl- und Caprinsäure. Die oft verwendete Bezeichnung "Neutralöl" hat die Substanz durch ihre geruchs- und geschmacksneutralen Eigenschaften erhalten. Als Zusatz in Cremes und Salben wirkt sie spreitungs- und penetrationsfördemd. Ebenso erfolgt der Einsatz als öliges Vehikel in Lösungen und Suspensionen. Im Bereich der parenteralen Ernährung dient es als Energielieferant [6].

### Glycerol

Glycerol (Glc, Mᵣ = 92,09) ist eine klare, fast farblose, sirupartige, stark hygroskopische Flüssigkeit von süßem Geschmack. Glc ist ungiftig und in Konzentrationen bis 30 % nicht hautreizend. Die Substanz dient als Emolliens sowie als Feuchthaltemittel in Emulsionen, Hydrogelen oder Lösungen und bewirkt eine bessere Streichfähigkeit von O/W-Emulsionen.

### Propylenglykol

Propylenglykol (PG, Mᵣ = 76,1) ist eine klare, farblose, nahezu geruchslose, viskose und hygroskopische Flüssigkeit von süßlichem Geschmack. Die Substanz ist weder haut- noch schleimhautreizend und hat penetrationsbeschleunigende und in Konzentrationen ab 15 % konservierende Eigenschaften. Es findet Anwendung als Solvens oder Cosolvens sowie in Konzentrationen über 5 % als Feuchthaltemittel.

### 2. Herstellung der Formulierungen

Für die Herstellung der Mikroemulsionen (MEs) wurden zuerst die Tensidmischung und die wässrige Phase durch Vermischen der Einzelkomponenten bereitgestellt. Die benötigten Mengen Tensidgemisch und Öl wurden in eine Fantaschale eingewogen und verrührt. Nach anteiliger Zugabe der wässrigen Phase wurde mit einem Pistill bis zum Klarwerden gerührt.
Vitaminhaltige ME's entstehen durch Anreiben der vorgelegten Substanzen mit ME und Auffüllen auf die erforderliche Endmassen.

### 3. Scale-up

Die Herstellung von 1 kg ME erfolgt mit Hilfe des Stephan-Rührwerkes UMC Electronic (Fassungsvermögen 5 kg). Zunächst werden die Komponenten der wässrigen Phase in eine Fantaschale eingewogen und mit einem Pistill verrührt. Nach dem Vorlegen der Tenside in das Stephan-Rührwerk werden diese 2 min mit 300 U/min homogenisiert. Anschließend wird das Öl zugegeben und für 4 min mit 300 U/min unter einem Vakuum von 35 bar gerührt. Die vorbereitete wässrige Phase wird in drei Anteilen zu der Tensid/Öl-Mischung gegeben und jeder Anteil für 2 min bei 300 U/min unter Vakuum eingearbeitet. Abschließend bleibt das Vakuum für weitere 10 min bestehen, um die herstellungsbedingt vorhandenen Luftblasen aus der ME zu entfernen.

### 4. Vitamine-Analytik (HPLC)

Die HPLC wird mit einer Anlage der Firma "Waters", welche mit der Pumpe Delta 600, einem Säulenofen, dem Autosampler 717 plus und dem PAD 2996 ausgestattet ist, durchgeführt. Die Trennung der Analyte erfolgt dabei mit Hilfe einer RP-18-Säule.

In Tab. 2 sind die konstanten analytischen und in Tab. 3 die zu verändernden Parameter der für die gemeinsame Quantifizierung der Vitamine entwickelten Gradientenmethode dargestellt.

**Tab. 2: Konstante analytische Parameter der entwickelten Gradienten-Methode**

| | |
|---|---|
| **Stationäre Phase** | Nucleosil C18 120x4 mm ID, |
| | 5 µm, Macherey-Nagel |
| **Lösungsmittel** | Ethanol |
| | |
| **Injektionsvolumen** | 8 µl (12 µl für das MSMM) |
| **Retentionszeit** | |
| | 2,01 min (Dxp) |
| | 8,25 min (AscPal) |
| | 12,50 min (TocAc) |
| | |
| **Detektion** | |
| | UV-VIS-Detektor |
| | e = 200 (Dxp und TocAc) |
| | e = 243 (AscPal) |
| | |
| | |
| | |

**Tab. 3: Veränderliche analytische Parameter der entwickelten Gradienten-Methode**

| **Zeit** **[min]** | **Mobile Phase** **[4,72 mM H₃PO₄/ACN]** | **Flussrate** **[ml/min]** |
|---|---|---|
| 2,0 | 94:6 | 1,5 |
| 4,0 | 1:99 | 1,8 |
| 9,0 | 1:99 | 1,8 |
| 9,5 | 1:99 | 2,0 |
| 13,5 | 1:99 | 2,0 |
| 15,5 | 94:6 | 1,5 |
| 25,0 | 94:6 | 1,5 |

Die Wiederholpräzisionen für die Dreifachinjektion der Konzentrationen 6 µg/ml bzw. 100 µg/ml lagen bei 4 % bzw. 1.5 %

Zur Erstellung der Kalibriergeraden für die Gehaltsbestimmungen wurden Verdünnungen einer Stammlösung im Bereich von 6 µg/ml bis 100 µg/ml und für die Liberationsversuche zwischen 3 µg/ml und 50 µg/ml herangezogen. Die mittels linearer Regression erhaltenen Geradengleichungen wurden ab einem Korrelationskoeffizienten von 0,998 akzeptiert. Die Bestimmungsgrenze entspricht für alle drei Vitamine der niedrigsten beiden Kalibrierungen gemessenen Konzentration von 3 µg/ml.

### 5. Stabilität der Vitamine in Mikroemulsion

Die Stabilitätsuntersuchungen des Extraktes in der beschriebenen Mikroemulsion erfolgt bei Raumtemperatur. Bei einer Lagerung der Vitamine in der erfindungsgemäßen Mikroemulsion über einen Zeitraum von bis zu 6 Monaten zeigt sich keine signifikante Gehaltsänderung (Tab. 4).

**Tab. 4: Stabilitätsuntersuchungen der Vitamie in Mikroemulsion bei Raumtemperatur.**

| Wochen | Mikroemulsion |
|---|---|
| 0 | 100,00 ± 5,47 |
| 1 | 100,10 ± 4,97 |
| 4 | 100,73 ± 0,69 |
| 8 | 99,93 ± 2,82 |

### 6. Untersuchungen zur Toxizität (Keratinozyten-Modell)

Im Keratinozyten-Modell wird die HaCaT-Zellinie verwendet, eine immortalisierte Zellinie aus humanen Keratinozyten. Anhand der Kristallviolett-Färbung lässt sich nach Inkubation mit der zu untersuchenden Substanz die Anzahl der lebenden Zellen im Vergleich zur unbehandelten Kontrolle bestimmen. Nach 24stündiger Inkubation zeigen sich mit den untersuchten Konzentrationen keine toxischen Erscheinungen.

### 7. Untersuchungen zur Toxizität (HET-CAM-Modell)

Das HET-CAM-Modell (Hen's Egg Test - Chorioallantoic Membrane) stellt eine einfache, schnell durchführbare, kostengünstige und sehr empfindliche Methode für die Bewertung des toxischen Potentials von Substanzen in vivo dar. Die zu untersuchenden Substanzen werden in Lösung auf die stark vaskularisierte Chorio-Allantois-Membran befruchteter Hühnereier aufgebracht. Dieses System ist international etabliert und zählt nicht als Tierversuch. Es zeigt sich also in beiden Modellen eine sehr gute Verträglichkeit der Substanzen in den untersuchten Konzentrationen.

### 8. Biopharmazeutische Untersuchungen [Klinische Studie (K42/06-ZKSD)]

Eine doppelblinde, randomisierte, vehikel- und referenzkontrollierte Studie mit kombinierter Anwenderpräferenztestung zur Verträglichkeit von 5 topischen dermatologischen Präparationen im Rahmen einer Epikutanen Verträglichkeitstestung (EVT) am hautgesunden Probanden verlief positiv.
Diese Studie erfolgte im Rahmen einer epikutanen Verträglichkeitstestung (EVT) am hautgesunden Probanden Die Daten wurden zentral per EDV umfasst und ausgewertet.
Die Dateneingabe erfolgte doppelt durch mindestens zwei voneinander unabhängige Personen mit anschließendem Datenvergleich per EDV. Als Software zur statistischen Auswertung wurden SIGMA STAT Windows Vers. 3.00 und MS EXCEL verwendet. Die statistische Planung, die Erstellung des Randomisierungsplans, sowie die Auswertung erfolgten durch den Studienbiometriker.
Zur biometrischen Auswertung wurde eine deskriptive Darstellung der qualitativen und quantitativen Absolutwerte erstellt. Weiterhin wurde darauf verwiesen, dass die Ergebnisse der vorliegenden Studie nicht für regulatorische Zwecke verwendet werden sollen und deshalb auf eine statistische Fallzahlschätzung und Auswertung verzichtet wurde.
Die Studie wurde wie im Prüfplan aufgeführt durchgeführt. Es wurden insgesamt 10 Probanden eingeschlossen. Insgesamt wurden 10 Probanden dem gesamten Studienprocedere unterzogen. Unerwünschte Ereignisse traten nicht auf. In die Studie wurden 6 Frauen und 4 Männer (n=10), die die klinischen und anamnestischen Einschlusskriterien erfüllten eingeschlossen. Alle Probanden wurden dem vollständigen Studienablauf unterzogen. Die rekrutierten Patienten hatten ein Durchschnittsalter von 27,9 Jahren (min 22 Jahre, max 33 Jahre), ein Durchschnittsgewicht von 71,6 kg (min 56 kg; max 91 kg) sowie eine Durchschnittsgröße von 177,7 cm (min 167 cm; max 198 cm).

### Hauptzielparameter Klinischer Score zur Verträglichkeit

Zusammenfassend lässt sich sagen, dass alle Prüfpräparate im Vergleich zum Leerfeld häufiger eine leichte Zunahme des Scores zeigen. Unterschiede zwischen den Prüfpräparaten ergeben sich nicht.

### Anwenderpräferenzen

Die subjektive Bewertung der Testpräparate ergab für die Prüfpräparate uneinheitliche Ergebnisse mit Bezug auf die einzelnen Parametergruppen:
***Konsistenz:*** alle Prüfpräparate werden im Vergleich zu den Referenzpräparaten etwas schlechter bewertet
***Hautgefühl beim Auftragen:*** hier zeigen sich keine Unterschiede
***Hautgefühl nach dem Auftragen:*** für Mandelöl zeigt sich hier ein deutlicher Nachteil beim Einziehen in die Haut; Apomix-C, Dex, E wird nachteilig in Bezug auf Hautgefühl bewertet;
***Duft:*** hier zeigen sich keine Unterschiede
***Gesamturteil:*** hier zeigen sich tendenziell Vorteile für Apomix-E und Apomix-Vehikel

### Nebenzielparameter Erythem (Erythrometrie)

Zusammenfassend lässt sich feststellen, dass im Vergleich zum unbehandelten Testareal keine Änderungen der Erythrometriewerte erkennbar sind.
Folgend ist ein klinischer Score zur Verträglichkeit dargestellt:

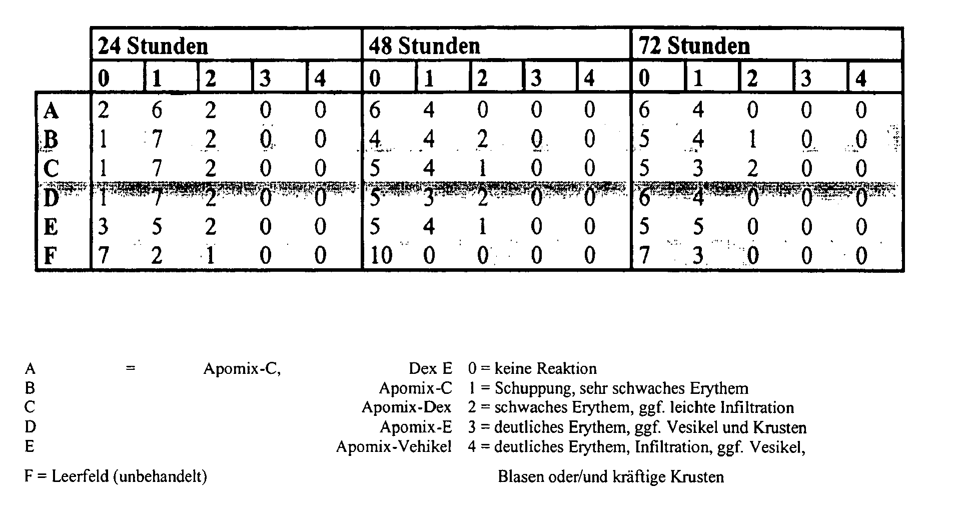

### Anwenderpräferenzen

### 1. Konsistenz

Optimum = 4,0

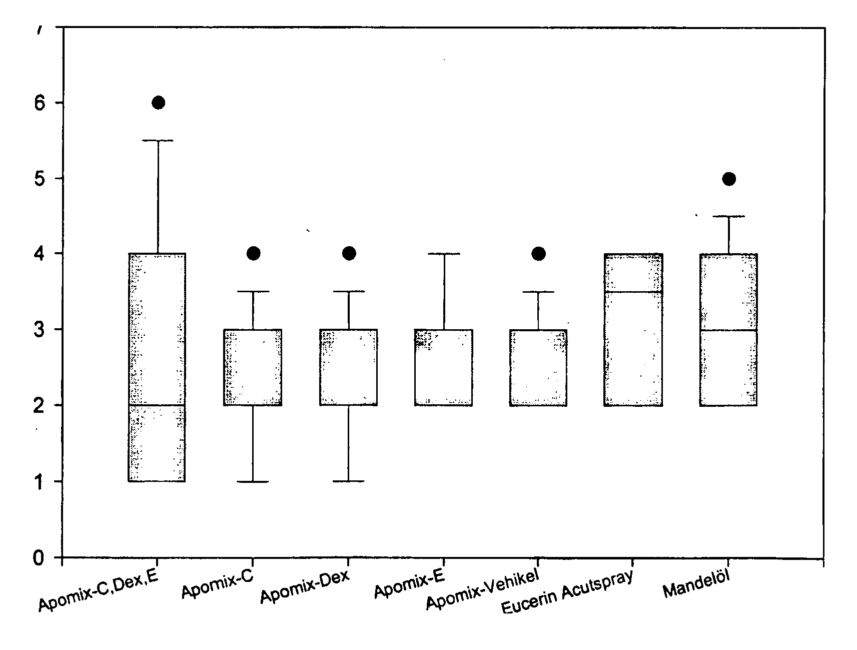

### 2. Hautgefühl beim Auftragen Das Produkt lässt sich gut verteilen

Optimum = 7,0

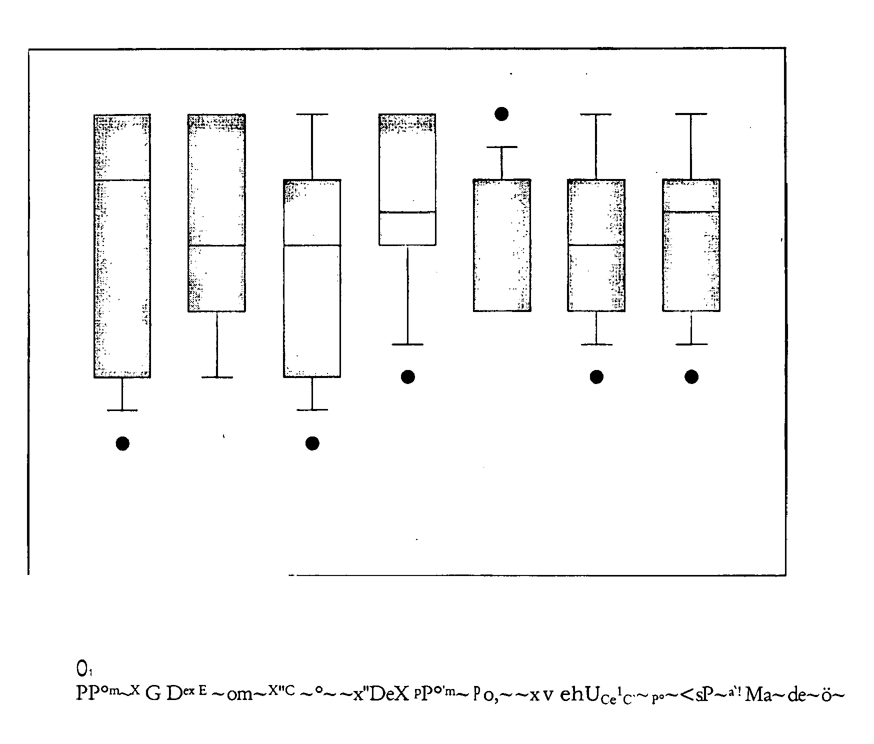

### Das Produkt fühlt sich reichhaltig/gehaltvoll an.

Optimum = 7,0

### 3. Hautgefühl nach dem Auftragen

### Das Produkt zieht schnell ein.

Optimum = 7,0
Die Haut fühlt sich angenehm gepflegt, glatt und weich an.
Optimum = 7,0

### Die Haut ist klebrig.

Optimum = 1,0
Das Produkt ist vollständig eingezogen.
Optimum = 7,0

### Das Hauptgefühl

Samtig/seidig = 1
Geschmeidig/weich = 2
Glatt=3Fettig= 4 Ölig=5
Stumpf = 6 Schmierig = 7

| Häufigkeiten: | | | | | | | |
|---|---|---|---|---|---|---|---|
| | 1 | 2 | 3 | 4 | 5 | 6 | 7 |
| **Apomix C, Dex, E** | 0 | 2 | 1 | 4 | 3 | 0 | 0 |
| **Apomix C** | 0 | 2 | 1 | 3 | 1 | 2 | 1 |
| **Apomix Dex** | 0 | 1 | 3 | 3 | 0 | 1 | 2 |
| **Apomix E** | 0 | 4 | 2 | 1 | 2 | 1 | 0 |
| **Apomix - Vehikel** | 1 | 1 | 4 | 2 | 0 | 0 | 2 |
| **Eucerin Akutspray** | 2 | 1 | 6 | 1 | 0 | 0 | 0 |
| **Mandelöl** | 0 | 2 | 1 | 2 | 4 | 0 | 1 |

### 4. Duft

Den Duft des Produktes empfinde ich als störend.
Optimum = 1,0

### 5. Gesamturteil zum Präparat

Insgesamt gesehen gefällt mir das Produkt sehr gut. Optimum = 7,0

### Erythrometrie

Erythrometrie nach 24 Stunden

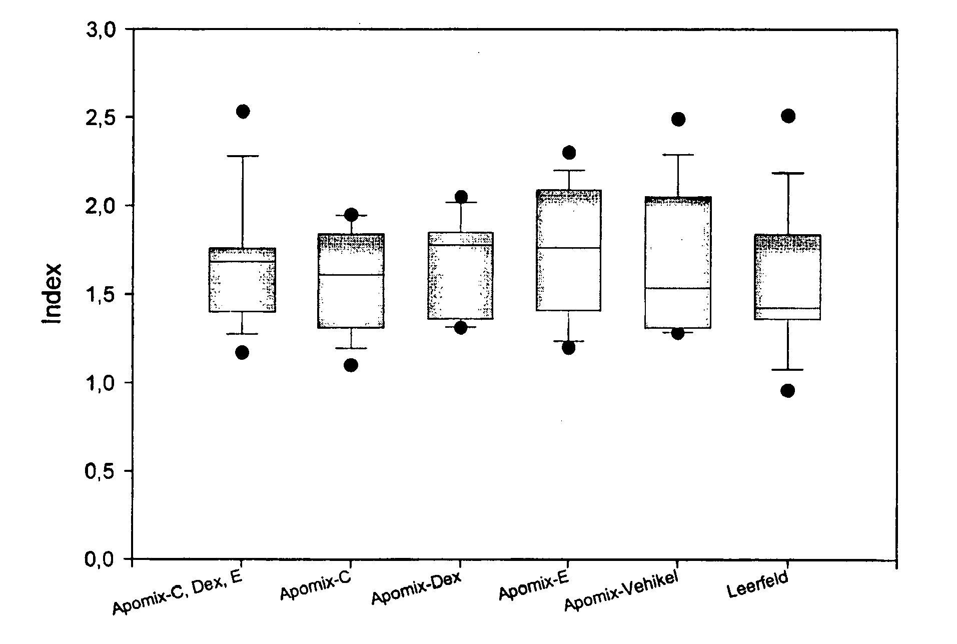

Erythrometrie nach 48 Stunden

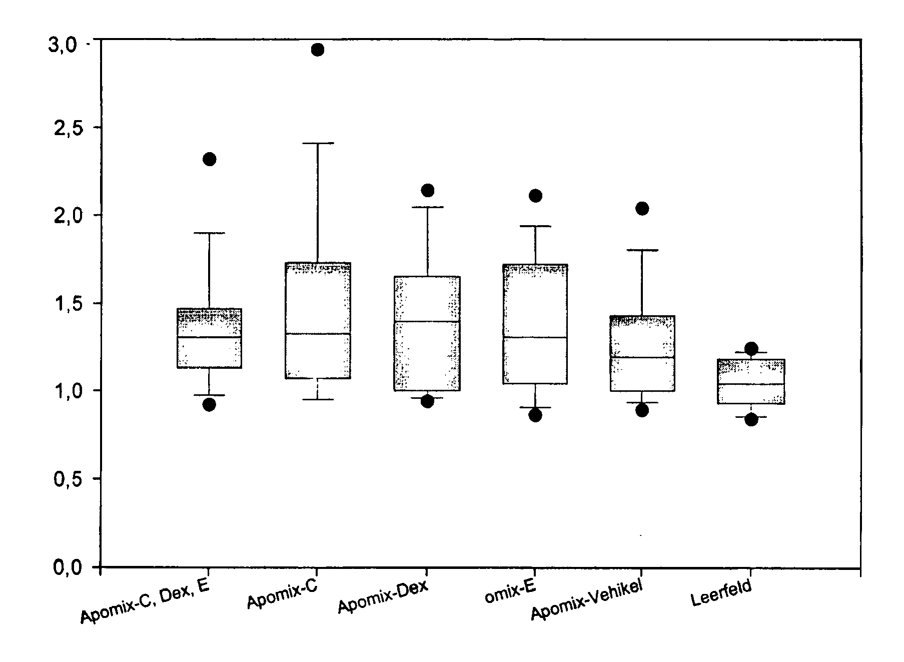

Alle in der Beschreibung, den Ausführungsbeispielen und den nachfolgenden Ansprüchen dargestellten Merkmale können sowohl einzeln als auch in beliebiger Kombination miteinander erfindungswesentlich sein.

## Patentansprüche

1. Kolloidales Trägersystem umfassend eine Tensid / Cotensid - Mischung, eine hydrophile Phase und eine lipophilen Komponente.

2. Kolloidales Trägersystem gemäß Anspruch 1, **dadurch gekennzeichnet, dass** die hydrophile Phase eine Mischung von Propylenglycol und Wasser ist.

3. Kolloidales Trägersystem gemäß Anspruch 1, **dadurch gekennzeichnet, dass** die lipophile Komponente Pelemol® BIP ist.

4. Kolloidales Trägersystem gemäß Anspruch 1, **dadurch gekennzeichnet, dass** es 20% Tagat O2/Synperonic PE/L 101 (2:3), 5% Myritol G 318 und 75% Glycerol/Propylenglycol/Wasser (0,3:1:1) enthält.

5. Kolloidales Trägersystem gemäß Anspruch 1, **dadurch gekennzeichnet, dass** es 20% Tagat O2/Synperonic PE/L 101 (2:3), 5% Myritol G 318 und 75% Glycerol/Propylenglycol/Wasser (0,15:1:1) enthält.

6. Verfahren zur Herstellung eines kolloidalen Trägersystems gemäß einem oder mehrerer der voran stehenden Ansprüche, **dadurch gekennzeichnet, dass** nichtionische Tenside, sowie lipophile und hydrophile Grundstoffe und Mischungen zusammengeführt werden, so dass sich die Mikroemulsionen spontan aus den Einzelbestandteilen ausbildet.

7. Verfahren zur Herstellung eines kolloidalen Trägersystems gemäß Anspruch 6, bei dem
• zur Vorbereitung die Komponenten der wässrigen Phase in eine Fantaschale eingewogen und mit einem Pistill verrührt werden
• nach dem Vorlegen der Tenside in ein Rührwerk werden diese 2 min mit 300 U/min homogenisiert
• anschließend wird das Öl zugegeben und für 4 min mit 300 U/min unter einem Vakuum von 35 bar gerührt
• die vorbereitete wässrige Phase wird in drei Anteilen zu der Tensid/Öl-Mischung gegeben und jeder Anteil für 2 min bei 300 U/min unter Vakuum eingearbeitet
• abschließend bleibt das Vakuum für weitere 10 min bestehen.

8. Verwendung eines kolloidalen Trägersystems gemäß einem oder mehrerer der voran stehenden Ansprüche zur dermalen (topischen) Applikation von schlecht resorbierbaren Vitaminen.

9. Verwendung eines kolloidalen Trägersystem gemäß einem oder mehrerer der voran stehenden Ansprüche zur dermalen (topischen) Applikation von Ascorbinsäure, Biotin, Retinsäure, Thiamin, Calciferol, Tokopherol, Dexpanthenol, D,L-α-Tocopherolacetat oder Ascorbylpalmitat.
